# EUROPEAN PATENT APPLICATION

(11) **EP 1 953 659 A1**
(43) Date of publication of application: **06.08.2008**
(21) Application number: 07101398.1
(22) Date of filing: 30.01.2007
(51) Int. Cl.: G06F 19/00

(54) **A method for effecting computer implemented decision-support in prescribing a drug therapy**

(71) Applicant: Daintel ApS, 1150 Copenhagen K (DK)
(72) Inventor: Dybdahl, Lars B., 3310, Ølsted (DK)
(74) Representative: Plougmann & Vingtoft A/S

(57) **Abstract**

The method uses a rules database comprising a number of associated rules for prescribing the drug for treatment, where each rule indicates a prescription of the drug suitable for a specific set of patient data. Drug data and patient data are entered into the rules database. The database provides an output of a prescription suitable for the therapy.

## Description

This invention relates to computer implemented decision-support in prescribing a drug therapy of a patient having a disease or a discomfort.

### Terminology used in this specification

### Prescription

A prescription contains the specification needed by e.g. a nurse to be able to administer drugs to a patient. This information comprises a number of smaller pieces of information.

### Indication

The indication is the medical reason for making a prescription. For instance, the drug Paracetamol may be prescribed for the indication head-ache.

### Drug

Throughout the current specification the term "drug" is meant to comprise an active substance or composition for the treatment, having any physical form such as tablets, powder, liquid or other form, its trade name or trade names, etc.

### Decision parameters

The set of decision parameters is the machine-readable information that can enable a computer to provide a suggestion for a prescription. Decision parameters include patient data and drug data and other relevant information.

### Procedure, rules database

A procedure is a rules database optionally combined with additional information. A rules database is a set of data that delivers information based on one or more lookups. Lookup is done using decision parameters as input to the rules database.

### Table

A table is a representation of a data structure with a plurality of fields (also referred to as cells), where each field is identified by a set of one or more coordinates. A table can comprise one or more sub-tables, which can be visually presented together or separately.

When a diagnosis has been made of a patient having a disease or a discomfort a drug must be selected for treating the patient. It is usually the responsibility of the physician or other medical personnel to select the drug.

US 7 010 431 B2 discloses a method for effecting computer-implemented decision-support in the selection of a drug therapy of patients having a viral disease such as HIV. A rules database is provided and patient data is entered including genotype data on the viral genome of the viral disease. The rules database comprises a number of associated rules for each available drug for treatment of the viral disease. Each rule indicates the suitability of the drug for treatment of a specific viral genotype. The patient data is entered into the rules database, the database providing an output of drugs suitable for therapy. The drugs suitable for therapy are displayed in a ranking in accordance with their suitability indication, for selection. The drug for use in the method of the present invention can also be selected using this method.

When a drug has been selected, e.g. as described above, for the treatment a prescription must be made for how to administer the drug to the patient. A prescription may include dosage of the drug, the frequency, duration etc. Several factors influence the prescription, in particular the patient data comprising e.g. gender, age, weight and clinical data and other observations. Many hospitals and clinics have standard operation procedures for the prescription of drugs used in the hospital or clinic. Such procedures are often in written form and describe verbally how patient data and other factors and conditions influence the prescription. However, such procedures are often not complete and may possibly also vary form one hospital or clinic to another. Often the procedures may also have to be supplemented with the knowledge and experience of the physician. Thus, the standard prescription obtained using the procedure may be adjusted to the actual patient.

It is therefore desirable to have a complete rules database, e.g. in the form of a table, which for each drug gives one prescription for each possible combination of patient data. However, it is a large job to establish such a rules database.

Furthermore, each possible prescription must be approved by a responsible physician or a local or national medical advisory board, which is also a large job. It is therefore also desirable to have the complete rules database represented in a form that can be handled without losing the overview, e.g. in the form of a look-up table. It is therefore also desirable to reduce the size of such a rules database in order to improve the overview without losing information. The size of the rules database should also be minimized for technical and practical reasons, e.g. in order to:
• Reduce the required storage space in computers handling it,
• Enable it to be printed on fewer pieces of paper
• Enable easier error checking.

The choice of a prescription could then be made using a look-up table, i.e.
1. Find or calculate the decision parameters, and
2. Look up in the table and find the prescription.
Individual adjustments of the prescription can still be made, mainly because the automated decision parameters may or may not have included all situations.

An obvious way to implement a rules database is to make it provide a predefined set of information on a specific lookup mechanism.

Example of a full rules database in the form of a table:

**Table 1**

| Life threatening? | Focus? | Drug | Amount | How long |
|---|---|---|---|---|
| Yes | Unknown | Acme Anti-Infection | 2 tablets, | 3 days |
| | | 500mg tablet | 3 times per day | |
| Yes | A | Acme Anti-Infection | 2 tablets, | 7 days |
| | | 500mg tablet | 3 times per day | |
| Yes | B | Acme Anti-Infection | 2 tablets, | 8 days |
| | | 500mg tablet | 3 times per day | |
| Yes | C | Acme Anti-Infection | 2 tablets, | 10 days |
| | | 500mg tablet | 3 times per day | |
| No | Unknown | Acme Anti-Infection | 1 tablet, | 3 days |
| | | 500mg tablet | 2 times per day | |
| No | A | Acme Anti-Infection | 1 tablet, | 7 days |
| | | 500mg tablet | 2 times per day | |
| No | B | Acme Anti-Infection | 1 tablet, | 8 days |
| | | 500mg tablet | 2 times per day | |
| No | C | Acme Anti-Infection | 1 tablet, | 10 days |
| | | 500mg tablet | 2 times per day | |

In the above table 1 there is one row for each of the possible combinations of the decision parameters. The prescribing physician should find the particular row in the table which contains the actual set of decision parameters, DP (here: Life threatening Focus and Drug). The prescription information, PI (here: Amount, How long) should then be taken from the PI cells in the same row. In many real life situations, however, there will be many decision parameters, and the size and complexity of the table increase exponentially with the number of decision parameters, since all possible combinations should be covered and each combination of decision parameters and the corresponding prescription information should appear in one and the same row. For large and complex tables it is a correspondingly big task to approve the information in the table and its use in the prescription process, and still, in daily use such large tables require great care and accuracy in order to avoid misreading and prescription errors. Thus, it would be advantageous to have a rules database of reduced size and improved readability.

Consequently, and in accordance with the invention, we will define, that:
• The table may require multiple lookups, i.e. all information should not necessarily be in one row, and
• Each lookup can provide different sets of information, and that we do not know what kind of information will be given before the lookup has been made.

This is implemented by:
• Allowing fields of information, i.e. table cells, to be empty
• Combining the information from multiple lookups into one prescription, i.e. the prescription should possibly be found in fields which are not all in one row of the table.

### Example: A reduced rules database for the anti-infection drug in table 1:

**Table 2**

| Life threatening? | Focus? | Drug | Amount | How long |
|---|---|---|---|---|
| | | Acme Anti-Infection 500mg tablet | | |
| | Unknown | | | 3 days |
| | A | | | 7 days |
| | B | | | 8 days |
| | C | | | 10 days |
| Yes | | | 2 tablets, | |
| | | | 3 times per day | |
| No | | | 1 tablet, | |
| | | | 2 times per day | |

Table 2 contains the same information as table 1, however the size is reduced and it is easier to read. The following explanation of table 2 may be useful:
• The decision parameters for this table are two questions: "Focus?" and "Life threatening?" The possible answers are A/B/C/Unknown and Yes/No.
• The first two columns are lookup columns. Find all rows where those two columns match the decision parameters. If a field is empty, consider it a match. This means that the first line in the table is always a match, no matter what the decision parameters are. As you can see, this new table has fewer rows (7 instead of 8) and much fewer fields with values.

The following improvements were made as part of the research into making a prescription system:
• Assembling prescription information from various pieces of information from a rules database, where the lookup must be made before it is known which lookup provides which information.
• Storing the rules database in this modular way.

The following extra features are included in the system:
The rules database may contain values that are not directly copied to the final prescription, but may activate certain calculations. For instance, we can have a dose factor column. If this value is set in a row that is found by a lookup, it is multiplied to the dose found by other lookups before the final prescription is obtained. This makes it easy to specify, e.g. that "In case of kidney trouble, only use 50 % of the dose".

In the following a general algorithm for constructing the reduced rules database will be described. Let's say we have this rules database (DP=Decision Parameter, PI=Prescription Information, empty fields mean that it's a match).

Repeat the following process:
1. Find a PI value that is produced as a result of a specific set of DP values, which have cell content in common, which they do not have in common with other sets of DP values. For instance, in the above example, PI1 is always 222 for the DP sets starting with A. All these sets have DP1 = A in common, and no other DP sets have DP1 = A.
2. Add a line to the table, where the common DP values are present. The other DP values should be empty. Only copy the common PI value to this new row.
3. Remove that specific PI value from the rows that have these sets of DP values.
4. Remove rows that have no PI values.
Naturally, in this process rows and columns of the table may be interchanged. In the following an illustrative example will be given.

### Example:

This is the full and detailed list of prescriptions, based on the decision parameters Indication, AdmChoice and Kidney:

**Table 3**

| **Indication** | **Adm Choice?** | **Kidney?** | **DruglD** | **Adm Route** | **AdmMethod** | **Dose** | **Unit** | **Repetition** | **Max/day** | **Duration** |
|---|---|---|---|---|---|---|---|---|---|---|
| A | 1 | C | 28101599394 | IV | Infusion 15-30 min | 20 | mg/kg | x2 | 160 | 3 days |
| A | 1 | Z | 28101599394 | IV | Infusion 15-30 min | 5 | mg/kg | x1 | 20 | 3 days |
| A | 1 | X | 28101599394 | IV | Infusion 15-30 min | 20 | mg/kg | x2 | 120 | 3 days |
| A | 1 | N | 28101599394 | IV | Infusion 15-30 min | 20 | mg/kg | x3 | 160 | 3 days |
| A | 1 | Y | 28101599394 | IV | Infusion 15-30 min | 10 | mg/kg | x2 | 160 | 3 days |
| A | 2 | C | 28101599394 | IV | Injection 3-5 min | 20 | mg/kg | x2 | 160 | 3 days |
| A | 2 | Z | 28101599394 | IV | Injection 3-5 min | 5 | mg/kg | x1 | 20 | 3 days |
| A | 2 | X | 28101599394 | IV | Injection 3-5 min | 20 | mg/kg | x2 | 120 | 3 days |
| A | 2 | N | 28101599394 | IV | Injection 3-5 min | 20 | mg/kg | x3 | 160 | 3 days |
| A | 2 | Y | 28101599394 | IV | Injection 3-5 min | 10 | mg/kg | x2 | 160 | 3 days |
| B | 1 | C | 28101599394 | IV | Infusion 15-30 min | 20 | mg/kg | x2 | 160 | 7 days |
| B | 1 | Z | 28101599394 | IV | Infusion 15-30 min | 5 | mg/kg | x1 | 20 | 7 days |
| B | 1 | X | 28101599394 | IV | Infusion 15-30 min | 20 | mg/kg | x2 | 120 | 7 days |
| B | 1 | N | 28101599394 | IV | Infusion 15-30 min | 20 | mg/kg | x3 | 160 | 7 days |
| B | 1 | Y | 28101599394 | IV | Infusion 15-30 min | 10 | mg/kg | x2 | 160 | 7 days |
| B | 2 | C | 28101599394 | IV | Injection 3-5 min | 20 | mg/kg | x2 | 160 | 7 days |
| B | 2 | Z | 28101599394 | IV | Injection 3-5 min | 5 | mg/kg | x1 | 20 | 7 days |
| B | 2 | X | 28101599394 | IV | Injection 3-5 min | 20 | mg/kg | x2 | 120 | 7 days |
| B | 2 | N | 28101599394 | IV | Injection 3-5 min | 20 | mg/kg | x3 | 160 | 7 days |
| B | 2 | Y | 28101599394 | IV | Injection 3-5 min | 10 | mg/kg | x2 | 160 | 7 days |
| C | 1 | C | 28101599394 | IV | Infusion 15-30 min | 20 | mg/kg | x2 | 160 | 10 days |
| C | 1 | Z | 28101599394 | IV | Infusion 15-30 min | 5 | mg/kg | x1 | 20 | 10 days |
| C | 1 | X | 28101599394 | IV | Infusion 15-30 min | 20 | mg/kg | x2 | 120 | 10 days |
| C | 1 | N | 28101599394 | IV | Infusion 15-30 min | 20 | mg/kg | x3 | 160 | 10 days |
| C | 1 | Y | 28101599394 | IV | Infusion 15-30 min | 10 | mg/kg | x2 | 160 | 10 days |
| C | 2 | C | 28101599394 | IV | Injection 3-5 min | 20 | mg/kg | x2 | 160 | 10 days |
| C | 2 | Z | 28101599394 | IV | Injection 3-5 min | 5 | mg/kg | x1 | 20 | 10 days |
| C | 2 | X | 28101599394 | IV | Injection 3-5 min | 20 | mg/kg | x2 | 120 | 10 days |
| C | 2 | N | 28101599394 | IV | Injection 3-5 min | 20 | mg/kg | x3 | 160 | 10 days |
| C | 2 | Y | 28101599394 | IV | Injection 3-5 min | 10 | mg/kg | x2 | 160 | 10 days |

Then we identify common content for Indications A, B and C, and move the associated data to new rows:

**Table 4**

| **Indication** | **Adm Choice?** | **Kidney?** | **DrugID** | **Adm Route** | **AdmMethod** | **Dose** | **Unit** | **Repetition** | **Max/day** | **Duration** |
|---|---|---|---|---|---|---|---|---|---|---|
| A | 1 | C | | | Infusion 15-30 min | 20 | | x2 | 160 | |
| A | 1 | Z | | | Infusion 15-30 min | 5 | | x1 | 20 | |
| A | 1 | X | | | Infusion 15-30 min | 20 | | x2 | 120 | |
| A | 1 | N | | | Infusion 15-30 min | 20 | | x3 | 160 | |
| A | 1 | Y | | | Infusion 15-30 min | 10 | | x2 | 160 | |
| A | 2 | C | | | Injection 3-5 min | 20 | | x2 | 160 | |
| A | 2 | Z | | | Injection 3-5 min | 5 | | x1 | 20 | |
| A | 2 | X | | | Injection 3-5 min | 20 | | x2 | 120 | |
| A | 2 | N | | | Injection 3-5 min | 20 | | x3 | 160 | |
| A | 2 | Y | | | Injection 3-5 min | 10 | | x2 | 160 | |
| B | 1 | C | | | Infusion 15-30 min | 20 | | x2 | 160 | |
| B | 1 | Z | | | Infusion 15-30 min | 5 | | x1 | 20 | |
| B | 1 | X | | | Infusion 15-30 min | 20 | | x2 | 120 | |
| B | 1 | N | | | Infusion 15-30 min | 20 | | x3 | 160 | |
| B | 1 | Y | | | Infusion 15-30 min | 10 | | x2 | 160 | |
| B | 2 | C | | | Injection 3-5 min | 20 | | x2 | 160 | |
| B | 2 | Z | | | Injection 3-5 min | 5 | | x1 | 20 | |
| B | 2 | X | | | Injection 3-5 min | 20 | | x2 | 120 | |
| B | 2 | N | | | Injection 3-5 min | 20 | | x3 | 160 | |
| B | 2 | Y | | | Injection 3-5 min | 10 | | x2 | 160 | |
| C | 1 | C | | | Infusion 15-30 min | 20 | | x2 | 160 | |
| C | 1 | Z | | | Infusion 15-30 min | 5 | | x1 | 20 | |
| C | 1 | X | | | Infusion 15-30 min | 20 | | x2 | 120 | |
| C | 1 | N | | | Infusion 15-30 min | 20 | | x3 | 160 | |
| C | 1 | Y | | | Infusion 15-30 min | 10 | | x2 | 160 | |
| C | 2 | C | | | Injection 3-5 min | 20 | | x2 | 160 | |
| C | 2 | Z | | | Injection 3-5 min | 5 | | x1 | 20 | |
| C | 2 | X | | | Injection 3-5 min | 20 | | x2 | 120 | |
| C | 2 | N | | | Injection 3-5 min | 20 | | x3 | 160 | |
| C | 2 | Y | | | Injection 3-5 min | 10 | | x2 | 160 | |
| A | | | 28101599394 | IV | | | mg/kg | | | 3 days |
| B | | | 28101599394 | IV | | | mg/kg | | | 7 days |
| C | | | 28101599394 | IV | | | mg/kg | | | 10 days |

Then we identify data in common for each admchoice, and move that to new rows:

**Table 5**

| **Indication** | **Adm Choice?** | **Kidney?** | **DrugID** | **Adm Route** | **AdmMethod** | **Dose** | **Unit** | **Repetition** | **Max/day** | **Duration** |
|---|---|---|---|---|---|---|---|---|---|---|
| A | 1 | C | | | | 20 | | x2 | 160 | |
| A | 1 | Z | | | | 5 | | x1 | 20 | |
| A | 1 | X | | | | 20 | | x2 | 120 | |
| A | 1 | N | | | | 20 | | x3 | 160 | |
| A | 1 | Y | | | | 10 | | x2 | 160 | |
| A | 2 | C | | | | 20 | | x2 | 160 | |
| A | 2 | Z | | | | 5 | | x1 | 20 | |
| A | 2 | X | | | | 20 | | x2 | 120 | |
| A | 2 | N | | | | 20 | | x3 | 160 | |
| A | 2 | Y | | | | 10 | | x2 | 160 | |
| B | 1 | C | | | | 20 | | x2 | 160 | |
| B | 1 | Z | | | | 5 | | x1 | 20 | |
| B | 1 | X | | | | 20 | | x2 | 120 | |
| B | 1 | N | | | | 20 | | x3 | 160 | |
| B | 1 | Y | | | | 10 | | x2 | 160 | |
| B | 2 | C | | | | 20 | | x2 | 160 | |
| B | 2 | Z | | | | 5 | | x1 | 20 | |
| B | 2 | X | | | | 20 | | x2 | 120 | |
| B | 2 | N | | | | 20 | | x3 | 160 | |
| B | 2 | Y | | | | 10 | | x2 | 160 | |
| C | 1 | C | | | | 20 | | x2 | 160 | |
| C | 1 | Z | | | | 5 | | x1 | 20 | |
| C | 1 | X | | | | 20 | | x2 | 120 | |
| C | 1 | N | | | | 20 | | x3 | 160 | |
| C | 1 | Y | | | | 10 | | x2 | 160 | |
| C | 2 | C | | | | 20 | | x2 | 160 | |
| C | 2 | Z | | | | 5 | | x1 | 20 | |
| C | 2 | X | | | | 20 | | x2 | 120 | |
| C | 2 | N | | | | 20 | | x3 | 160 | |
| C | 2 | Y | | | | 10 | | x2 | 160 | |
| A | | | 28101599394 | IV | | | mg/kg | | | 3 days |
| B | | | 28101599394 | IV | | | mg/kg | | | 7 days |
| C | | | 28101599394 | IV | | | mg/kg | | | 10 days |
| | 1 | | | | Infusion 15-30 min | | | | | |
| | 2 | | | | Injection 3-5 min | | | | | |

Then we identify data for each kidney choice:

**Table 6**

| **Indication** | **Adm Choice?** | **Kidney?** | **DrugID** | **Adm Route** | **AdmMethod** | **Dose** | **Unit** | **Repetition** | **Max/day** | **Duration** |
|---|---|---|---|---|---|---|---|---|---|---|
| A | 1 | C | | | | | | | | |
| A | 1 | Z | | | | | | | | |
| A | 1 | X | | | | | | | | |
| A | 1 | N | | | | | | | | |
| A | 1 | Y | | | | | | | | |
| A | 2 | C | | | | | | | | |
| A | 2 | Z | | | | | | | | |
| A | 2 | X | | | | | | | | |
| A | 2 | N | | | | | | | | |
| A | 2 | Y | | | | | | | | |
| B | 1 | C | | | | | | | | |
| B | 1 | Z | | | | | | | | |
| B | 1 | X | | | | | | | | |
| B | 1 | N | | | | | | | | |
| B | 1 | Y | | | | | | | | |
| B | 2 | C | | | | | | | | |
| B | 2 | Z | | | | | | | | |
| B | 2 | X | | | | | | | | |
| B | 2 | N | | | | | | | | |
| B | 2 | Y | | | | | | | | |
| C | 1 | C | | | | | | | | |
| C | 1 | Z | | | | | | | | |
| C | 1 | X | | | | | | | | |
| C | 1 | N | | | | | | | | |
| C | 1 | Y | | | | | | | | |
| C | 2 | C | | | | | | | | |
| C | 2 | Z | | | | | | | | |
| C | 2 | X | | | | | | | | |
| C | 2 | N | | | | | | | | |
| C | 2 | Y | | | | | | | | |
| A | | | 28101599394 | IV | | | mg/kg | | | 3 days |
| B | | | 28101599394 | IV | | | mg/kg | | | 7 days |
| C | | | 28101599394 | IV | | | mg/kg | | | 10 days |
| | 1 | | | | Infusion 15-30 min | | | | | |
| | 2 | | | | Injection 3-5 min | | | | | |
| | | C | | | | 20 | | x2 | 160 | |
| | | Z | | | | 5 | | x1 | 20 | |
| | | X | | | | 20 | | x2 | 120 | |
| | | N | | | | 20 | | x3 | 160 | |
| | | Y | | | | 10 | | x2 | 160 | |

As you can see, there are now a lot of rows that can be removed:

**Table 7**

| **Indication** | **Adm Choice?** | **Kidney?** | **DrugID** | **Adm Route** | **AdmMethod** | **Dose** | **Unit** | **Repetition** | **Max/day** | **Duration** |
|---|---|---|---|---|---|---|---|---|---|---|
| A | | | 28101599394 | IV | | | mg/kg | | | 3 days |
| B | | | 28101599394 | IV | | | mg/kg | | | 7 days |
| C | | | 28101599394 | IV | | | mg/kg | | | 10 days |
| | 1 | | | | Infusion 15-30 min | | | | | |
| | 2 | | | | Injection 3-5 min | | | | | |
| | | C | | | | 20 | | x2 | 160 | |
| | | Z | | | | 5 | | x1 | 20 | |
| | | X | | | | 20 | | x2 | 120 | |
| | | N | | | | 20 | | x3 | 160 | |
| | | Y | | | | 10 | | x2 | 160 | |

This process has reduced table 3 with 30 rows and all fields filled, to table 7 with 10 rows and lot of empty fields and with the same information as in the original table 3.

### Method for reading the reduced table:

Go through all parts of the table. Check, for all parts of the table, whether the given DP values are in accordance with the basis for deciding on the prescription, and if they are, take out the shown PI values. Combine all PI values found to a prescription.

The computer implemented method of the invention is suitable for using any of the rules databases or tables shown here. The number of fields having data in the table in figure 7 is greatly reduced. Consequently, the overview is clearly improved. The reduced tables have the technical advantage of requiring less storage space and less transmission time when e.g. being downloaded or transmitted. The reduced table 7 is more simple and easier to read and is likely to reduce the number of errors in the prescription process. For larger tables, these advantages are more pronounced.

## Claims

1. A method for effecting computer implemented decision-support in prescribing a drug therapy of a patient having a disease or a discomfort, using a preselected drug for the therapy, the method comprising
• using a rules database,
• providing an input of patient data including the disease or the discomfort,
• wherein the rules database comprises a number of associated rules for prescribing the preselected drug for treatment of the disease or the discomfort, each rule indicating a prescription of the drug suitable for a specific set of patient data,
• entering data on the preselected drug into the rules database,
• entering the patient data into the rules database,
• the database providing an output of a prescription suitable for the therapy.

2. A computer programmed to carry out the method of claim 1.

3. A computer program carrier comprising a computer program in a format downloadable by a computer, comprising a computer program executable by the computer for causing the computer to carry out the method of claim 1.

4. A method for reducing the size of a rules database for decision-support in prescribing a drug therapy of a patient having a disease or a discomfort, the rules database comprising a table having rows and columns with one or more columns of decision parameters (DP) representing patient data, and one or more columns of prescription information (PI) representing elements of the prescription, where each row contains a set of decision parameters (DP) and a set of prescription information (PI) corresponding to the set of decision parameters (DP) in the same row, the method comprising
- finding a prescription information (PI) value that corresponds to a specific set of decision parameter (DP) values having cell content in common, which they do not have in common with other sets of decision parameter (DP) values,
- adding a row to the table, where the common decision parameter (DP) values are present and leaving the remaining decision parameter (DP) values empty,
- copying the common prescription information (PI) value to the added row,
- removing the prescription information (PI) value from the rows that have these sets of DP values, and
- removing rows that have no prescription information (PI) values.

5. A rules database obtained using the method of claim 4.

6. A computer programmed to carry out the method of claim 4.

7. A computer program carrier comprising a computer program in a format downloadable by a computer, comprising a computer program executable by the computer for causing the computer to carry out the method of claim 4.
